# EUROPEAN PATENT APPLICATION

(11) **EP 4 095 126 A1**
(43) Date of publication of application: **30.11.2022**
(21) Application number: 21740758.4
(22) Date of filing: 15.01.2021
(51) Int. Cl.: C07C 323/52, C07C 319/20, C07C 323/53, C07C 323/54, C07F 7/18, G01T 1/161

(54) **FATTY ACID DERIVATIVE LABELED BY POSITRON-EMITTING NUCLIDE**

(30) Priority: 17.01.2020 JP 2020005535
(71) Applicant: Astellas Pharma Inc., Chuo-ku Tokyo 103-8411 (JP)
(72) Inventor: MURAKAMI, Yoshihiro, Tokyo 103-8411 (JP); FUSHIKI, Hiroshi, Tokyo 103-8411 (JP); YONEZAWA, Koichi, Tokyo 103-8411 (JP)
(74) Representative: Gille Hrabal Partnerschaftsgesellschaft mbB Patentanwälte
(86) International application number: PCT/JP2021/001154
(87) International publication number: WO 2021/145403

(57) **Abstract**

[Problem] To provide a labeled fatty acid derivative for diagnostic imaging that enables the quantification of myocardial fatty acid metabolic activity. [Solution] The inventors, engaging in diligent research into methods that enable the quantification of fatty acid metabolic activity, discovered that a labeled fatty acid derivative represented by formula (I), in which [¹⁸F] has been substituted at a specific position of a long-chain carboxylic acid compound containing a sulfur atom, or a salt thereof, has good accumulation in the myocardium, and enables imaging of fatty acid metabolic activity via positron emission tomography (PET). Therefore, the labeled fatty acid derivative according to the present invention can be used as a radiotracer for swift and noninvasive quantification of myocardial fatty acid metabolic activity, diagnostic imaging of heart diseases such as ischemic heart disease, diagnostic imaging of the therapeutic effects yielded by a heart disease therapeutic agent, and so forth.

## Description

### Technical Field

The present invention relates to a fatty acid derivative labeled with a positron-emitting radionuclide or a salt thereof. Further, the present invention relates to a composition for diagnostic imaging containing the labeled fatty acid derivative or a salt thereof, and a kit for preparing the same.

### Background Art

The heart requires a large amount of adenosine triphosphate (ATP) to pump blood throughout the body, most of which is produced by oxidative metabolism in mitochondria. Various substrates such as fatty acids, glucose, and the like are metabolized in the myocardium, but in a state in which oxygen is fully distributed to the myocardium, about 60% to 90% of the required ATP is produced by β-oxidation of fatty acids. On the other hand, it has been found that in an ischemic state, the rate of oxidative metabolism decreases and the rate of ATP production by anaerobic metabolism increases (Expert Rev. Cardiovasc. Ther. 2007; 5 (6): 1123-1134.). Therefore, the quantification of fatty acid metabolic activity in the myocardium is one of the effective means for diagnosing heart disease.

This quantification of fatty acid metabolic activity is carried out by positron emission tomography (PET) or single photon emission computed tomography (SPECT) using a labeled long-chain fatty acid derivative as a radiolabeled tracer. As a radiolabeled tracer used for SPECT, 15-(p-[¹²³I]iodophenyl)-3(R,S)-methylpentadecanoic acid (hereinafter, abbreviated as [¹²³I]BMIPP) represented by the following formula has been reported (Non-Patent Document 1), and has been actually used in clinical practice for diagnosing heart disease.

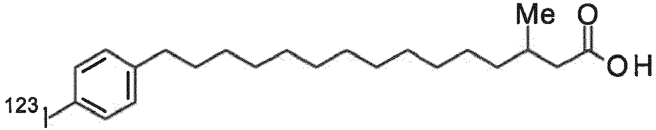

(In the formula, Me represents a methyl group. The same shall apply hereinafter.)

On the other hand, as a radiolabeled tracer used for PET, 16-[¹⁸F]fluoro-4-thiahexadecanoic acid represented by the following formula (also referred to as 16-[¹⁸F]fluoro-4-thiapalmitic acid; hereinafter, abbreviated as [¹⁸F]FTP) has been known (Patent Document 1).

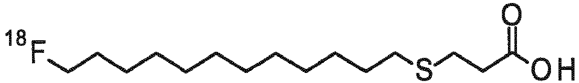

Similarly, as the radiolabeled tracer used for PET, 3-{[(5Z)-14-(¹⁸F]fluorotetradec-5-en-1-yl]sulfanyl}propanoic acid represented by the following formula, which has improved accumulation in the heart compared to the [¹⁸F]FTP, (also referred to as 18-[¹⁸F]fluoro-4-thiaoleic acid; hereinafter, abbreviated as [¹⁸F]FTO) has been reported (Non-Patent Document 2).

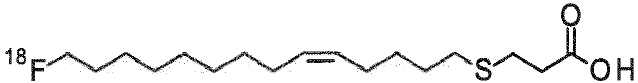

However, [¹²³I]BMIPP used as a tracer for diagnostic imaging by SPECT has a problem of low sensitivity and resolution, and although [¹⁸F]FTP and [¹⁸F]FTO as the tracer used for diagnostic imaging by PET are superior in SPECT using the [¹²³I]BMIPP, further improvement in sensitivity and resolution is required for more accurate diagnosis.

Moreover, Patent Document 2 published after the priority date of the present application describes 3-{[(5Z)-3-[¹⁸F]fluorotetradec-5-en-1-yl]sulfanyl}propanoic acid (also referred to as 7-[¹⁸F]fluoro-4-thiaoleic acid; hereinafter, abbreviated as 7-[¹⁸F]FTO) represented by the following formula, as the radiolabeled tracer used for PET.

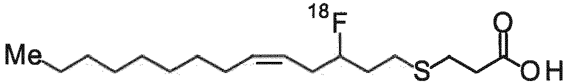

### Related Art

### Patent Document

[Patent Document 1] International Publication No. 2000/063216
[Patent Document 2] International Publication No. 2020/017620

### Non-Patent Document

[Non-Patent Document 1] Eur. J. Nucl. Med., 1989, 15, 341-345
[Non-Patent Document 2] J. Nucl. Med., 2010, 51(8), 1310-1317

### Disclosure of Invention

### Problems to Be Solved by the Invention

There is a strong desire for the development of a radiolabeled tracer that has selective accumulation to the heart and improved sensitivity and resolution and enables accurate diagnosis of heart disease.

### Means for Solving the Problems

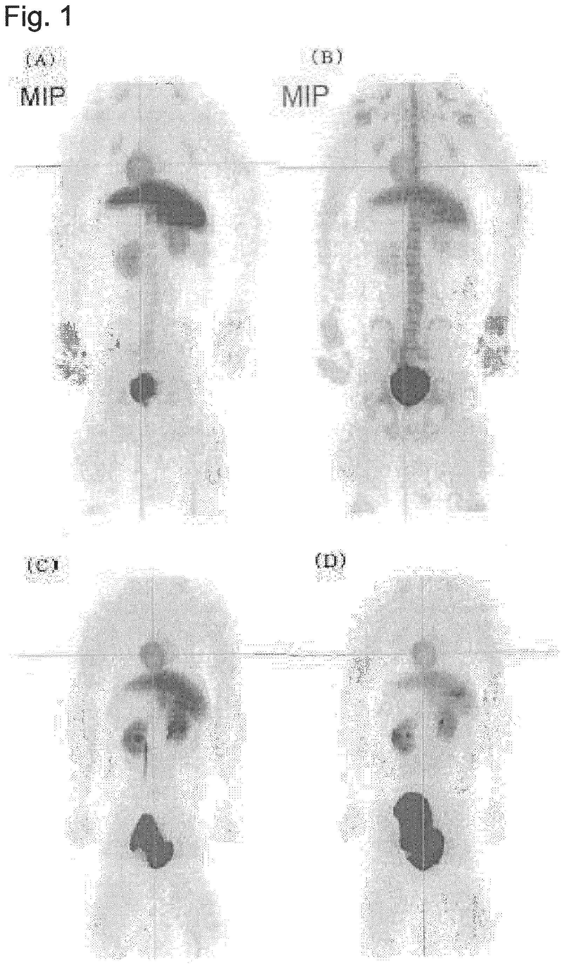

In PET images using [¹⁸F]FTO which is a known PET tracer, accumulation of [¹⁸F]fluorine is confirmed not only in the heart but also in the bone. Thus, it is considered that the background value is increased and the sensitivity and resolution are decreased (for example, refer to PET image with [¹⁸F]FTO in FIG. 1).

In further investigations, β-oxidation and ω-oxidation are present in the oxidative metabolism pathway of long-chain fatty acids. In the ω-oxidation, fatty acids are metabolized from the terminal side away from the carboxyl group to generate [¹⁸F]fluoride ions in the process of metabolism of [¹⁸F]FTO, and [¹⁸F]sodium fluoride has accumulation to the bone. Therefore, it is assumed that the [¹⁸F]fluoride ions generated in the process of metabolism of [¹⁸F]FTO may be the cause of non-specific accumulation to the bone.

As a result of conducting intensive investigations for the purpose of producing a labeled fatty acid derivative that is less likely to generate [¹⁸F] fluoride ions by metabolism, the present inventors and the like have found that unexpectedly, a labeled fatty acid derivative represented by the formula (I) in which [¹⁸F]fluorine is substituted on the carbon atom bonded via two carbon atoms to a sulfur atom of long-chain carboxyl acid compound containing a sulfur atom can be used as a radiolabeled tracer which has low accumulation of [¹⁸F]fluoride ions to the bone compared to the conventional [¹⁸F]FTO, is selectively accumulated in the heart, and enables imaging of fatty acid metabolism in the myocardium with high sensitivity, and thus have completed the present invention. It is presumed that by introducing [¹⁸F]fluorine into a specific substitution position, the metabolic stability against ω-oxidation from the terminal side away from the carboxyl group is improved, the generation of [¹⁸F]fluoride ions is suppressed, and as a result, the accumulation in the bone is reduced.

That is, the present invention relates to a labeled fatty acid derivative represented by the formula (I) or a salt thereof.

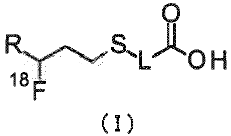

(In the formula, L is C₂₋₄ alkylene, R is C₆₋₁₄ alkyl, and the C₆₋₁₄ alkyl may have one to three double bonds. In addition, three continuous carbon atoms in the C₆₋₁₄ alkyl may integrally form a cyclopropane ring, provided that, a case where R is (2Z)-undec-2-en-1-yl group is excluded.)

Unless otherwise specified, in a case where a symbol in a chemical formula in the specification is also used in another chemical formula, the same symbol has the same meaning.

In addition, the present invention also relates to
(1) a composition for diagnostic imaging containing a labeled fatty acid derivative represented by the formula (I) or a salt thereof, and a pharmaceutically acceptable carrier, particularly a composition for diagnostic imaging of heart disease, and more particularly a composition for diagnostic imaging of ischemic heart disease,
(2) use of the labeled fatty acid derivative represented by the formula (I) or a salt thereof for the production of a composition for diagnostic imaging of heart disease, particularly ischemic heart disease,
(3) use of the labeled fatty acid derivative represented by the formula (I) or a salt thereof for diagnostic imaging of heart disease, particularly ischemic heart disease,
(4) a labeled fatty acid derivative represented by the formula (I) or a salt thereof for use in diagnostic imaging of heart disease, particularly ischemic heart disease,
(5) a method for diagnostic imaging of heart disease, particularly ischemic heart disease, including administering a detectable amount of the labeled fatty acid derivative represented by the formula (I) or a salt thereof to a subject,
(6) an intermediate compound, which can be converted into the labeled fatty acid derivative represented by the formula (I) or a salt thereof, or a salt thereof,
(7) a method for producing the labeled fatty acid derivative represented by the formula (I) or a salt thereof from the intermediate compound or salt thereof according to (6), and
(8) a kit for diagnostic imaging for preparing the composition for diagnostic imaging according to (1) including the intermediate compound or salt thereof according to (6).

A "subject" is a human or other animal which is in need of such a diagnosis, and in certain embodiment, is a human which is in need of such a diagnosis.

### Effects of the Invention

The labeled fatty acid derivative represented by the formula (I) according to the present invention or a salt thereof does not cause an increase in background value due to accumulation in the bone compared to [¹⁸F]FTO which is a known labeled fatty acid, has excellent accumulation to the myocardium and enables imaging of fatty acid metabolic activity by PET. Therefore, the labeled fatty acid derivative represented by the formula (I) according to the present invention or a salt thereof can be used as a radiolabeled tracer for sorting heart disease patients, diagnostic imaging of the therapeutic effect of a therapeutic drug for heart disease, and the like.

### Brief Description of Drawings

FIG. 1 shows PET images in which a compound ([¹⁸F]FTO) of Reference Example 15 and a compound of Example 6 are administered to normal cynomolgus monkeys in Example 8, respectively. PET images 60 minutes and 180 minutes after administration of [¹⁸F]FTO are shown in (A) and (B), respectively. In addition, PET images 60 minutes and 180 minutes after administration of the compound of Example 6 are shown in (C) and (D), respectively.

### Embodiments for Carrying Out the Invention

Hereinafter, the present invention will be described in detail.

In the specification, the "reagent for labeling ¹⁸F" is a reagent containing [¹⁸F]fluoride ions, and examples thereof include tetra-n-butylammonium [¹⁸F]fluoride (hereinafter, abbreviated as [¹⁸F]TBAF) and potassium [¹⁸F]fluoride (hereinafter, abbreviated as [¹⁸F]KF).

In the specification, the expression "may be substituted" means "unsubstituted" or one or more arbitrary substituents to bind thereto.

In the specification, the "C₂₋₄ alkylene" is a linear or branched alkyl having 2 to 4 carbon atoms (hereinafter, abbreviated as C₂₋₄) and examples thereof include ethylene, trimethylene, tetramethylene, propylene, 1-methyltrimethylene, 2-methyltrimethylene, ethylethylene, 1,1-dimethylethylene, and 1,2-dimethylethylene. As one embodiment, the C₂₋₄ alkylene is ethylene, trimethylene, or tetramethylene, as another embodiment, ethylene or trimethylene, as still another embodiment, ethylene.

The "C₆₋₁₄ alkyl" is a linear or branched alkyl having 6 to 14 carbon atoms (C₆₋₁₄) and examples thereof include n-hexyl, n-heptyl, n-octyl, n-nonyl, n-decyl, n-undecyl, n-dodecyl, n-tridecyl, n-tetradecyl, isooctyl, 4-ethylheptyl, and 3,3-dimethyldecyl. As one embodiment, the C₆₋₁₄ alkyl is a linear C₆₋₁₄ alkyl, as another embodiment, n-octyl, n-nonyl, n-decyl, n-undecyl, or n-dodecyl, as still another embodiment, n-undecyl.

"The C₆₋₁₄ alkyl may have one to three double bonds" means that the C₆₋₁₄ alkyl includes the group in which the carbon-carbon single bond contained in the C₆₋₁₄ alkyl is substituted with a double bond. As one embodiment, R is n-undecyl having one to three double bonds. As another embodiment, R is (2E)-undec-2-en-1-yl, (2Z,5Z)-undeca-2,5-dien-1-yl, or (2Z,5Z,8Z)-undeca-2,5,8-trien-1-yl.

"Three continuous carbon atoms in the C₆₋₁₄ alkyl may integrally form a cyclopropane ring" means that the C₆₋₁₄ alkyl includes the group in which the carbon atoms positioned at both ends of three continuous carbon atoms contained in the C₆₋₁₄ alkyl are bonded by a single bond. As one embodiment, three continuous carbon atoms in the C₆₋₁₄ alkyl may each integrally form one to three cyclopropane ring. As another embodiment, R is (2-octylcyclopropyl)methyl.

The "lower alkyl" is a linear or branched C₁₋₆ alkyl and examples thereof include methyl, ethyl, n-propyl, isopropyl, n-butyl, isobutyl, s-butyl, t-butyl, n-pentyl, and n-hexyl. As one embodiment, the lower alkyl is C₁₋₄ alkyl, as another embodiment, methyl, ethyl, or t-butyl, and as still another embodiment, methyl.

The "leaving group" means a substituent that is eliminated by a nucleophilic substitution reaction, and examples thereof include sulfonyloxy group and halogen. However, there is no limitation thereto. Examples of the sulfonyloxy group include a p-toluenesulfonyloxy group, a p-nitrobenzenesulfonyloxy group, a methanesulfonyloxy group, and a trifluoromethanesulfonyloxy group, and as one embodiment, the sulfonyloxy group is a p-toluenesulfonyloxy group or a p-nitrobenzenesulfonyloxy group. Examples of halogen include Cl, Br, and I, and as one embodiment, the halogen is Br.

The "labeling" means that the specific atom in a compound is substituted with a radioactive isotope thereof. As one embodiment, the labeling means that the fluorine atom is substituted with ¹⁸F.

An embodiment of the labeled fatty acid derivative represented by the formula (I) or a salt thereof is shown in the following.

(1-1) The labeled fatty acid derivative or salt thereof, in which L is ethylene.

(1-2) The labeled fatty acid derivative or salt thereof, in which L is tetramethylene.

(2-1) The labeled fatty acid derivative or salt thereof, in which R is the C₆₋₁₄ alkyl.

(2-2) The labeled fatty acid derivative or salt thereof, in which R is the C₆₋₁₄ alkyl having one to three double bonds, provided that, a case where R is (2Z)-undec-2-en-1-yl is excluded.

(2-3) The labeled fatty acid derivative or salt thereof, in which R is the C₆₋₁₄ alkyl, and three continuous carbon atoms of the C₆₋₁₄ alkyl integrally form a cyclopropane ring.

(3) The labeled fatty acid derivative or salt thereof, which is a combination of the embodiment according to (1-1) or (1-2) and the embodiment according to any one of (2-1) to (2-3).

Further, an embodiment of the labeled fatty acid derivative represented by the formula (I) according to the present invention or a salt thereof is the following labeled fatty acid derivative or a salt thereof.
(1)3-{[(5Z,8Z,11Z)-3-[¹⁸F]fluorotetradeca-5,8,11-trien-1-yl]sulfanyl}propanoic acid
(2) 3-[(3-[¹⁸F]fluorododecyl)sulfanyl]propanoic acid
(3) 3-{[(5Z,8Z)-3-[¹⁸F]fluorotetradeca-5,8-dien-1-yl]sulfanyl}propanoic acid
(4) 3-[(3-[¹⁸F]fluorotetradecyl)sulfanyl]propanoic acid
(5) 3-{[(5E)-3-[¹⁸F]fluorotetradec-5-en-1-yl]sulfanyl}propanoic acid
(6) 3-{[3-[¹⁸F]fluoro-4-(2-octylcyclopropyl)butyl]sulfanyl}propanoic acid
(7) 5-[(3-[¹⁸F]fluoroundecyl)sulfanyl]pentanoic acid

The labeled fatty acid derivative represented by the formula (I) of the present invention or a salt thereof is used as a radiolabeled tracer for diagnostic imaging such as PET and the like. The labeled fatty acid derivative of the present invention has accumulation in the myocardium in a biological body, enables imaging of fatty acid metabolic activity by PET and a similar imaging method, and can be used for diagnostic imaging of heart disease, particularly, ischemic heart disease. It can also be used, in small animal tests, as in vivo diagnostic imaging means, for a planar positron imaging system (PPIS) which is a device that acquires planar accumulated images instead of tomographic images in addition to a PET system for small animals. Further, the labeled fatty acid derivative can be used for autoradiography, which is image analysis means for sections of extracted organs, and for evaluation of accumulation in extracted organs using a gamma counter.

The positron-emitting radionuclide used in the labeled fatty acid derivative of the present invention is ¹⁸F.

Generally, ¹⁸F is produced by an apparatus called a cyclotron. The ¹⁸F produced can be used to label the compound represented by the formula (I). A desired nuclide is obtained from a (ultra) small cyclotron installed in a facility to be used or the like, the labeled fatty acid derivative represented by the formula (I) of the present invention or a salt thereof is produced by a method known in the art, and thus a composition for diagnostic imaging can be prepared.

The labeled fatty acid derivative represented by the formula (I) of the present invention or a salt thereof may have an asymmetric center, and an enantiomer (optical isomer) based on the asymmetric center may be present. The compound represented by the formula (I) or a salt thereof includes any of isolated individual enantiomers such as an (R) body and an (S) body, and mixtures thereof (including a racemic mixture or a non-racemic mixture).

In addition, the labeled fatty acid derivative represented by the formula (I) of the present invention may form salts depending on conditions, and these salts are also included in the present invention. Here, examples of the salts include salts with metal positive ions such as sodium, potassium, magnesium, calcium, and aluminum, salts with organic bases such as methylamine, ethylamine, and ethanolamine, salts with various amino acids and amino acid derivatives such as acetyl leucine, lysine, and ornithine, and ammonium salts.

Further, the labeled fatty acid derivative represented by the formula (I) of the present invention or a salt thereof may be provided as a hydrate, a solvate, or a crystal polymorphic substance, and the present invention includes these forms.

The present invention includes an intermediate compound, which can be converted into the labeled fatty acid derivative represented by the formula (I) or a salt thereof, or a salt thereof. Examples of the "intermediate compound which can be converted into the labeled fatty acid derivative represented by the formula (I) or a salt thereof' include a compound represented by the formula (1g). Another embodiment includes a compound represented by the formula (If), which is a precursor of the compound represented by the formula (1g).

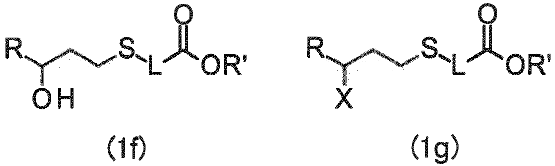

(In the formulae, R' is H or a lower alkyl group which may be substituted, and X is a leaving group. The same shall apply hereinafter.)

An embodiment of the compound represented by the formula (1g) is shown in the following.

(A-1) The compound in which R' is a lower alkyl which may be substituted.

(A-2) The compound in which R' is a lower alkyl.

(B-1) The compound in which L is C₂₋₄ alkylene.

(B-2) The compound in which L is ethylene.

(B-3) The compound in which L is tetramethylene.

(C-1) The compound in which R is C₆₋₁₄ alkyl, the C₆₋₁₄ alkyl may have one to three double bonds, and three continuous carbon atoms in the C₆₋₁₄ alkyl may integrally form a cyclopropane ring, provided that, a case where R is (2Z)-undec-2-en-1-yl group is excluded.

(C-2) The compound in which R is C₆₋₁₄ alkyl.

(C-3) The compound in which R is C₆₋₁₄ alkyl having one to three double bonds, provided that, a case where R is (2Z)-undec-2-en-1-yl is excluded.

(C-4) The compound in which R is C₆₋₁₄ alkyl, and three continuous carbon atoms in the C₆₋₁₄ alkyl integrally form a cyclopropane ring.

(D) The compound, which is a combination of the embodiment according to (A-1) to (C-4).

The present invention also includes a method for producing the labeled fatty acid derivative represented by the formula (I) or a salt thereof including a step of allowing a [¹⁸F]fluoride ion to react with the intermediate compound, which can be converted into the labeled fatty acid derivative represented by the formula (I) or a salt thereof, or a salt thereof.

Further, the present invention includes a kit for preparing a composition for diagnostic imaging containing at least the intermediate compound, which can be converted into the labeled fatty acid derivative represented by formula (I) or a salt thereof, or a salt thereof. An embodiment of the kit of the present invention is a kit for rapid synthesis of the labeled fatty acid derivative of the present invention. The kit includes a kit for preparing a composition for diagnostic imaging containing the intermediate compound, which can be converted into the labeled fatty acid derivative represented by the formula (I) or a salt thereof, or a salt thereof, and a reagent for labeling ¹⁸F, and is a kit containing other reagents, solvents, and the like if desired. The kit can be used for preparing the composition for diagnostic imaging of the present invention as required. As an embodiment, examples of the intermediate compound which can be converted into the labeled fatty acid derivative represented by the formula (I) or a salt thereof, or a salt thereof contained in the kit of the present invention include the compound represented by the formula (1g) or a salt thereof. In addition, the kit can also include instruments such as a reaction vessel, an apparatus for transferring isotopic material to the reaction vessel, a pre-packed separation column for separating a product from excess reactants, a shield, and the like as known in the art.

### (Production Method)

The labeled fatty acid derivative represented by the formula (I) or a salt thereof can be produced by applying various known synthetic methods using its basic structure or properties based on the type of substituent. At that time, depending on the type of functional group, in terms of production technology, it is effective to replace the functional group with an appropriate protective group (a group that can be easily converted to the functional group) at the stage from the starting material to the intermediate in some cases. Examples of such protective groups include the protective groups described in "Greene's Protective Groups in Organic Synthesis (5th Edition, 2014)" by P.G.M. Wuts, and the like, and depending on the reaction conditions thereof, the protective group may be appropriately selected and used. In such a method, a desired compound can be obtained by introducing the protective group to carry out the reaction, and then removing the protective group as required.

Hereinafter, a typical method for producing the labeled fatty acid derivative represented by the formula (I) or a salt thereof will be described. Each production method can also be carried out with reference to the references attached to the description. The production method of the present invention is not limited to the examples shown below.

The following abbreviations may be used in the specification.

DBU (registered trademark)=1,8-diazabicyclo[5.4.0]-7-undecene, DDQ=2,3-dichloro-5,6-dicyano-1,4-benzoquinone, EtOAc=ethyl acetate, HMPA=hexamethylphosphoric acid triamide, MeCN=acetonitrile, MeOH=methanol, Ph=phenyl, PyFluor=pyridine-2-sulfonylfluoride, TBAF=tetra-n-butylammonium fluoride, TBS=t-butyldimethylsilyl, TBDPS=t-butyldiphenylsilyl, TFA=trifluoroacetic acid, THF=tetrahydrofuran, Ts=p-toluenesulfonyl.

### (First step)

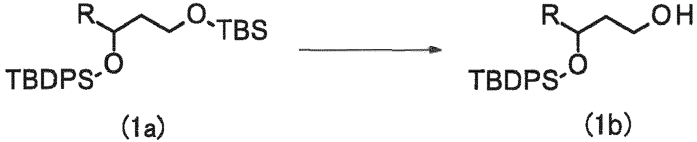

(In the formula, R is C₆₋₁₄ alkyl, the C₆₋₁₄ alkyl may have one to three double bonds, and three continuous carbon atoms in the C₆₋₁₄ alkyl may integrally form a cyclopropane ring, provided that, a case where R is (2Z)-undec-2-en-1-yl group is excluded. The same shall apply hereinafter.)

Compound (1b) can be produced by deprotecting a protective group TBS of compound (la).

Deprotection can be carried out using a method well known to those of skill in the art, and for example, deprotection can be carried out by performing stirring in a solvent inert to the reaction in the presence of a fluorine reagent or acid at -78°C to room temperature usually for 0.1 hours to 3 days. Examples of the fluorine reagent include TBAF and pyridine hydrofluoric acid, and acetic acid may be added at this time. Examples of the acid include hydrochloric acid and p-toluenesulfonic acid.

### (Second step)

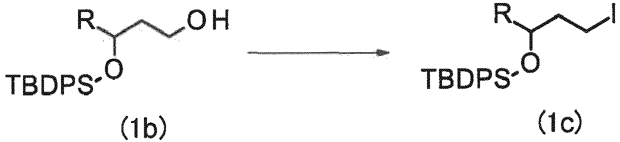

Compound (1c) can be produced by converting the hydroxyl group of compound (1b) into iodine by an iodination reaction.

The iodination reaction is carried out by stirring compound (1b) and iodine in a solvent inert to the reaction in the presence of triphenylphosphine and a base at ice cooling to room temperature usually for 0.1 hours to 3 days. Here, examples of the base include imidazole and triethylamine. Examples of the solvent include dichloromethane and THF.

### (Third step)

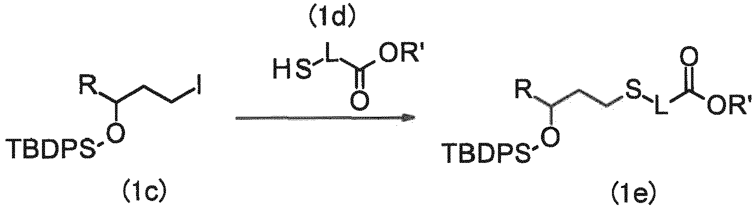

(In the formula, L is C₂₋₄ alkylene, and R' is H or a lower alkyl group which may be substituted. The same shall apply hereinafter.)

Compound (1e) can be produced from compound (1c) and compound (1d).

The reaction is carried out by stirring compound (1c) and compound (1d) in a solvent inert to the reaction in the presence of a base at ice cooling to room temperature for 0.1 hours to 3 days. Here, examples of the solvent include THF and 1,4-dioxane. Examples of the base include sodium hydride and potassium-t-butoxide.

### (Fourth step)

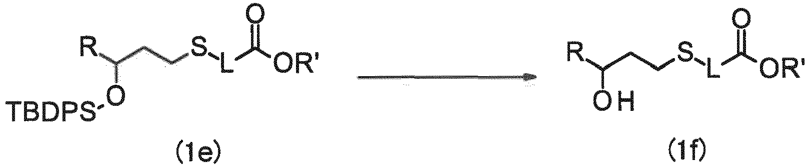

Compound (1f) can be produced by deprotecting a protective group TBDPS of compound (1e). Deprotection can be carried out in the same manner as in the first step.

### (Fifth step)

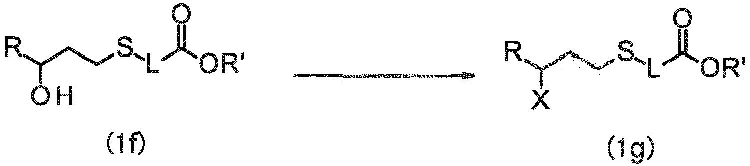

Compound (1g) can be produced by converting the hydroxyl group of compound (1f) into a leaving group.

In a case of producing the compound (1g) in which the leaving group is sulfonyloxy group, the reaction is carried out by stirring compound (1f) and a sulfonylating agent in a solvent inert to the reaction in the presence of a base at ice cooling to room temperature usually for 0.1 hours to 3 days. Here, examples of the sulfonylating agent include p-toluenesulfonyl chloride, p-nitrobenzenesulfonyl chloride, methanesulfonyl chloride, and trifluoromethanesulfonic anhydride. Examples of the solvent include dichloromethane and THF. Examples of the base include triethylamine and diisopropylethylamine. At this step, trimethylamine hydrochloride may be added.

In addition, in a case of producing the compound (1g) in which the leaving group is halogen, the reaction is carried out by stirring compound (1f) and a halogenating agent in a solvent inert to the reaction in the presence of triphenylphosphine at ice cooling to room temperature usually for 0.1 hours to 3 hours. Here, examples of the halogenating agent include carbon tetrabromide and iodine. Examples of the solvent include carbon tetrachloride, dichloromethane, and THF. At this time, a base such as imidazole, triethylamine, or the like may be added.

### (Sixth step)

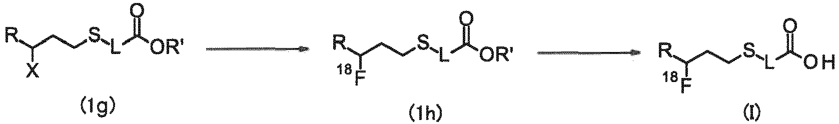

### (Sixth step-1)

Compound (1h) can be produced by, for example, causing a reaction of compound (1g) with a [¹⁸F]fluoride ion aqueous solution, tetra-n-butylammonium [¹⁸F]fluoride ([¹⁸F]TBAF), potassium [¹⁸F]fluoride, or the like, which is a reagent containing a positron-emitting radionuclide ¹⁸F produced by a cyclotron by a method well known to those skilled in the art, under heating.

### (Sixth step-2)

Compound (I) can be produced from compound (1h) by a method well known to those skilled in the art, and the reaction can be carried out by, for example, performing stirring in a solvent inert to the reaction in the presence of an aqueous base solution at ice cooling to 110°C usually for 0.1 hours to 3 days. Examples of the base include sodium hydroxide and potassium hydroxide. When R' is H, the sixth step-2 is omitted.

The isolation and purification of the compound represented by the formula (I) or a salt thereof produced in this manner are performed by applying ordinary chemical operations such as extraction, concentration, evaporation, crystallization, filtration, recrystallization, various types of chromatography, and the like.

Various isomers can be produced by selecting an appropriate starting compound, or can be separated by using a difference in physicochemical properties between the isomers. For example, optical isomers can be obtained by a general optical resolution method of a racemic body (for example, fractional crystallization leading to a diastereomeric salt with an optically active base or acid, chromatography using a chiral column and the like, and the like), and can be produced from an appropriate optically active starting compound.

A composition for diagnostic imaging according to the present invention can be produced by combining the labeled fatty acid derivative with at least one pharmaceutically acceptable carrier. The composition for diagnostic imaging of the present invention preferably has a dosage form suitable for intravenous administration, and is, for example, an injection for intravenous administration. Examples of the injection include those containing a sterile aqueous or non-aqueous solution, suspension, and emulsion. As the aqueous solvent, for example, distilled water for injection or physiological saline is included. Examples of the non-aqueous solvent include propylene glycol, polyethylene glycol, vegetable oils such as olive oil, alcohols such as ethanol, Polysorbate 80 (trade name), and the like. Such a composition may further contain a tonicity agent, a preservative, a wetting agent, an emulsifying agent, a dispersing agent, a stabilizing agent, or a solubilizing agent. These are sterilized by, for example, filtering in which these are filtered through a bacteria retentive filter, by being mixed with a germicide, or by irradiation. In addition, these can be used by being prepared as a sterile solid composition and dissolved or suspended in sterile water or a sterile solvent for injection before use. As one embodiment, the composition for diagnostic imaging of the present invention is an injection for intravenous administration. As another embodiment, the composition is an aqueous solution.

The composition for diagnostic imaging of the present invention can be used by adjusting the dose according to the imaging method (such as PET and the like) used, the type of disease, the age and state of the patient, the examination site, and the purpose of imaging. Although the composition for diagnostic imaging of the present invention is required to contain a detectable amount of the labeled fatty acid derivative, it is required to pay sufficient attention to the exposure dose of the patient. For example, the amount of the radioactivity of the composition for diagnostic imaging of the present invention labeled with ¹⁸F is about 1.85 to 740 megabecquerels (MBq), as one embodiment, the radioactivity is about 1.85 to 37 MBq, and as another embodiment, the radioactivity is about 37 to 740 MBq. The composition is administered once or in multiple time in a divided manner, or persistently instilled.

### Examples

Hereinafter, a method for producing the labeled fatty acid derivative of the present invention and the effect thereof will be described in more detail based on examples. The present invention is not limited to the compounds described in the following examples.

In addition, the following abbreviations may be used in Examples, Reference Examples and Tables below in some cases.

The following abbreviations represent the followings, respectively: Ex: Example number (The branch number indicates the step number in which the compound is obtained as a result in the example. For example, the compound whose Example number is Ex1-3 means that the compound is a compound obtained in the third step of Example 1), Ref: Reference example number, Str: Chemical structural formula, DAT: Physicochemical data, ESI+: m/z value in mass spectrometry (Ionization method ESI, representing [M + H]⁺ unless otherwise specified), ESI-: m/z value in mass spectrometry (Ionization method ESI, representing [M - H]⁻ unless otherwise specified), CI+: m/z value in mass spectrometry (Ionization method CI, representing [M + H]⁺ unless otherwise specified), NMR: δ value (ppm) of signal in 1H-NMR in CDCl₃, J: coupling constant, s: singlet, d: doublet, t: triplet, m: multiplet, and Ci: Curie, the unit of radioactivity (1 Ci = 3.7 × 10¹⁰ Bq).

For convenience, the concentration mol/L is represented by M. For example, a 1 M aqueous sodium hydroxide solution means a 1 mol/L aqueous sodium hydroxide solution.

### Example 1

Reference Example 8 shows a method for synthesizing 2,2,9,9,10,10-hexamethyl-3,3-diphenyl-5-[(2Z,5Z,8Z)-undeca-2,5,8-trien-1-yl]-4,8-dioxa-3,9-disilaundecane, which is the starting material in the first step.

First step: 2,2,9,9,10,10-hexamethyl-3,3-diphenyl-5-[(2Z,5Z,8Z)-undeca-2,5,8-trien-1-yl]-4,8-dioxa-3,9-disilaundecane (2.27 g) was dissolved in a mixture of THF (23 mL) and water (1.15 mL), p-toluenesulfonic acid monohydrate (375 mg) was added at room temperature, and the mixture was stirred at the same temperature overnight. Aqueous sodium bicarbonate and ethyl acetate were added, and the organic layer was separated, washed with a saturated brine, then dried over magnesium sulfate, and concentrated under reduced pressure. The residue was purified by silica gel column chromatography (EtOAc/n-hexane) to obtain (5Z,8Z,11Z)-3-{[t-butyl(diphenyl)silyl]oxy}tetradeca-5,8,11-trien-1-ol (1.83 g) as an oily substance.

Second step: Dichloromethane (90 mL) was added to triphenylphosphine (1.66 g) and dissolved under a nitrogen gas stream, iodine (1.61 g) and imidazole (0.54 g) were added, and the mixture was stirred at room temperature for 10 minutes. A dichloromethane (9 mL) solution of (5Z,8Z,11Z)-3-{[t-butyl(diphenyl)silyl]oxy}tetradeca-5,8,11-trien-1-ol (1.83 g) was added, and the mixture was stirred at room temperature for 30 minutes. An aqueous sodium thiosulfate solution was added, the mixture was stirred for 5 minutes, and chloroform and water were added to perform a liquid-separating operation. The organic layer was separated, then dried over magnesium sulfate, and concentrated under reduced pressure. N-Hexane was added to the residue, the precipitated solid substrate was removed through filtering, and the filtrate was purified by silica gel column chromatography (EtOAc/n-hexane) to obtain t-butyl{[(5Z,8Z,11Z)-1-iodotetradeca-5,8,11-trien-3-yl]oxy}diphenylsilane (1.73 g) as an oily substance.

Third step: A THF (35 mL) solution of methyl 3-sulfanyl propionate (0.49 mL) was ice-cooled under a nitrogen gas stream, and sodium hydride (60% dispersion in mineral oil, 180 mg) was added. The mixture was stirred at the same temperature for 30 minutes, a THF (8 mL) solution of t-butyl{[(5Z,8Z,11Z)-1-iodotetradeca-5,8,11-trien-3-yl]oxy}diphenylsilane (1.73 g) was added, and then the mixture was stirred at room temperature overnight. An ammonium chloride aqueous solution was added, and then water and ethyl acetate were added to perform a liquid-separating operation. The organic layer was separated, then washed with a saturated brine, dried over magnesium sulfate, and concentrated under reduced pressure. The residue was purified by silica gel column chromatography (EtOAc/n-hexane) to obtain methyl 3-{[(5Z,8Z,11Z)-3-{[t-butyl(diphenyl)silyl]oxy}tetradeca-5,8,11-trien-1-yl]sulfanyl}propanoate (1.65 g) as an oily substance.

Fourth step: Methyl 3-{[(5Z,8Z,11Z)-3-{[t-butyl(diphenyl)silyl]oxy}tetradeca-5,8,11-trien-1-yl]sulfanyl}propanoate (1.65 g) was dissolved in THF (33 mL) under a nitrogen gas stream, then ice-cooled. TBAF (1 M THF solution, 15 mL) was added, and the mixture was stirred at room temperature overnight. The mixture was diluted by adding ethyl acetate, then washed with water and a saturated brine, dried over magnesium sulfate, and then concentrated under reduced pressure. The residue was purified by silica gel column chromatography (EtOAc/n-hexane) to obtain methyl 3-{[(5Z,8Z,11Z)-3-hydroxytetradeca-5,8,11-trien-1-yl]sulfanyl}propanoate (0.62 g) as an oily substance.

Fifth step: Methyl 3-{[(5Z,8Z,11Z)-3-hydroxytetradeca-5,8,11-trien-1-yl]sulfanyl}propanoate (120 mg) was dissolved in dichloromethane (2.4 mL) under a nitrogen gas stream. Triethylamine (0.13 mL) and trimethylamine hydrochloride (35 mg) were added, and cooled at a bath temperature of -5°C. p-Toluenesulfonyl chloride (105 mg) was added little by little, then the mixture was stirred at the same temperature for 30 minutes, and aqueous sodium bicarbonate and chloroform were added, followed by extraction with chloroform. The organic layer was dried over magnesium sulfate, and then concentrated under reduced pressure. The residue was purified by silica gel column chromatography (EtOAc/n-hexane) to obtain methyl 3-({(5Z,8Z,11Z)-3-[(4-methylbenzene-1-sulfonyl)oxy]tetradeca-5,8,11-trien-1-yl} sulfanyl)propanoate (141 mg) as an oily substance.

Sixth step: After adsorbing a [¹⁸F]fluoride ion aqueous solution obtained from a cyclotron onto a pre-conditioned anion exchange resin (Sep-Pak (registered trademark) Light QMA), a target material was eluted with a mixed solution of 75 mM tetra-n-butylammonium hydrogencarbonate (0.20 mL) and MeCN (0.8 mL). The eluent was concentrated under reduced pressure at 140°C, MeCN (1 mL) was added, and the mixture was concentrated under reduced pressure at 140°C. MeCN (1 mL) was added again, and the mixture was concentrated under reduced pressure at 120°C to obtain [¹⁸F]TBAF. Here, a MeCN (0.8 mL) solution of methyl 3-({(5Z,8Z,11Z)-3-[(4-methylbenzene-1-sulfonyl)oxy]tetradeca-5,8,11-trien-1-yl}sulfanyl)propanoate (4 µL) was added and the mixture was heated at 130°C for 10 minutes. A 0.2 M aqueous potassium hydroxide solution (0.2 mL) was added to the reaction liquid, and the mixture was heated at 110°C for 4 minutes. A 50% MeCN aqueous solution (1.8 mL) and acetic acid (25 µL) were added to the reaction liquid and the mixture was purified by HPLC (0.005% TFA aqueous solution/0.005% TFA-MeCN solution (30:70), column: YMC-Pack Pro C18, S-5 µm, 10×250 mm, flow rate: 5 mL/min). Water (15 mL) was added to the obtained 3-{[(5Z,8Z,11Z)-3-[¹⁸F]fluorotetradeca-5,8,11-trien-1-yl]sulfanyl}propanoic acid fraction, and the solution was allowed to pass through Sep-Pak (registered trademark) Light C18, and the resin was washed with a 35% aqueous ethanol solution (5 mL). Subsequently, the target material was eluted with ethanol (1 mL), and then mixed with 1% ascorbic acid aqueous solution (1 mL). An eluent obtained by passing 0.5% Tween (registered trademark) 80-saline (10 mL) through the resin, was mixed with the above eluent, and was allowed to pass through a sterile filter to obtain a solution containing 3-{[(5Z,8Z,11Z)-3-[¹⁸F]fluorotetradeca-5,8,11-trien-1-yl]sulfanyl}propanoic acid (66.0 mCi). The generation of the compound was confirmed by the retention time in HPLC being consistent with the time of the non-radiative labeled material shown in Reference Example 1.

### Example 2

Reference Example 9 shows a method for synthesizing 2,2,9,9,10,10-hexamethyl-5-nonyl-3,3-diphenyl-4,8-dioxa-3,9-disilaundecane, which is the starting material in the first step.

First step: 3-{[t-butyl(diphenyl)silyl]oxy}dodecan-1-ol was obtained in the same manner as in the first step of Example 1 except that 2,2,9,9,10,10-hexamethyl-5-nonyl-3,3-diphenyl-4,8-dioxa-3,9-disilaundecane was used as a starting material.

Second step: t-Butyl[(1-iodododecan-3-yl)oxy]diphenylsilane was obtained in the same manner as in the second step of Example 1 except that 3-{[t-butyl(diphenyl)silyl]oxy}dodecan-1-ol was used as a starting material.

Third step: Methyl 3-[(3-{[t-butyl(diphenyl)silyl]oxy}dodecyl)sulfanyl]propanoate was obtained in the same manner as in the third step of Example 1 except that t-butyl[(1-iodododecan-3-yl)oxy]diphenylsilane was used as a starting material.

Fourth step: Methyl 3-[(3-hydroxydodecyl)sulfanyl]propanoate was obtained in the same manner as in the fourth step of Example 1 except that methyl 3-[(3-{[t-butyl(diphenyl)silyl]oxy}dodecyl)sulfanyl]propanoate was used as a starting material.

Fifth step: Methyl 3-({3-[(4-methylbenzene-1-sulfonyl)oxy]dodecyl}sulfanyl)propanoate was obtained in the same manner as in the fifth step of Example 1 except that methyl 3-[(3-hydroxydodecyl)sulfanyl]propanoate was used as a starting material.

Sixth step: 3-[(3-[¹⁸F]fluorododecyl)sulfanyl]propanoic acid was obtained in the same manner as in the sixth step of Example 1 except that methyl 3-({3-[(4-methylbenzene-1-sulfonyl)oxy]dodecyl}sulfanyl)propanoate was used as a starting material. The generation of the compound was confirmed by the retention time in HPLC being consistent with the time of non-radiative labeled material shown in Reference Example 2.

### Example 3

Reference Example 10 shows a method for synthesizing 2,2,9,9,10,10-hexamethyl-3,3-diphenyl-5-[(2Z,5Z)-undeca-2,5-dien-1-yl]-4,8-dioxa-3,9-disilaundecane, which is the starting material in the first step.

First step: (5Z,8Z)-3-{[t-butyl(diphenyl)silyl]oxy}tetradeca-5,8-dien-1-ol was obtained in the same manner as in the first step of Example 1 except that 2,2,9,9,10,10-hexamethyl-3,3-diphenyl-5-[(2Z,5Z)-undeca-2,5-dien-1-yl]-4,8-dioxa-3,9-disilaundecane was used as a starting material.

Second step: t-Butyl{[(5Z,8Z)-1-iodotetradeca-5,8-dien-3-yl]oxy}diphenylsilane was obtained in the same manner as in the second step of Example 1 except that (5Z,8Z)-3-{[t-butyl(diphenyl)silyl]oxy}tetradeca-5,8-dien-1-ol was used as a starting material.

Third step: Methyl 3-{[(5Z,8Z)-3-{[t-butyl(diphenyl)silyl]oxy}tetradeca-5,8-dien-1-yl]sulfanyl}propanoate was obtained in the same manner as in the third step of Example 1 except that t-butyl{[(5Z,8Z)-1-iodotetradeca-5,8-dien-3-yl]oxy}diphenylsilane was used as a starting material.

Fourth step: Methyl 3-{[(5Z,8Z)-3-hydroxytetradeca-5,8-dien-1-yl]sulfanyl}propanoate was obtained in the same manner as in the fourth step of Example 1 except that methyl 3-{[(5Z,8Z)-3-{[t-butyl(diphenyl)silyl]oxy}tetradeca-5,8-dien-1-yl]sulfanyl}propanoate was used as a starting material.

Fifth step: Methyl 3-({(5Z,8Z)-3-[(4-methylbenzene-1-sulfonyl)oxy]tetradeca-5,8-dien-1-yl}sulfanyl)propanoate was obtained in the same manner as in the fifth step of Example 1 except that methyl 3-{[(5Z,8Z)-3-hydroxytetradeca-5,8-dien-1-yl]sulfanyl}propanoate was used as a starting material.

Sixth step: 3- {[(5Z,8Z)-3-[¹⁸F]fluorotetradeca-5,8-dien-1-yl]sulfanyl}propanoic acid was obtained in the same manner as in the sixth step of Example 1 except that methyl 3-({(5Z,8Z)-3-[(4-methylbenzene-1-sulfonyl)oxy]tetradeca-5,8-dien-1-yl}sulfanyl)propanoate was used as a starting material. The generation of the compound was confirmed by the retention time in HPLC being consistent with the time of non-radiative labeled material shown in Reference Example 3.

### Example 4

Reference Example 11 shows a method for synthesizing 2,2,9,9,10,10-hexamethyl-3,3-diphenyl-5-undecyl-4,8-dioxa-3,9-disilaundecane, which is the starting material in the first step.

First step: 3-{[t-butyl(diphenyl)silyl]oxy}tetradecan-1-ol was obtained in the same manner as in the first step of Example 1 except that 2,2,9,9,10,10-hexamethyl-3,3-diphenyl-5-undecyl-4,8-dioxa-3,9-disilaundecane was used as a starting material.

Second step: t-Butyl[(1-iodotetradecan-3-yl)oxy]diphenylsilane was obtained in the same manner as in the second step of Example 1 except that 3-{[t-butyl(diphenyl)silyl]oxy}tetradecan-1-ol was used as a starting material.

Third step: Methyl 3-[(3-{[t-butyl(diphenyl)silyl]oxy}tetradecyl)sulfanyl]propanoate was obtained in the same manner as in the third step of Example 1 except that t-butyl[(1-iodotetradecan-3-yl)oxy]diphenylsilane was used as a starting material.

Fourth step: Methyl 3-[(3-hydroxytetradecyl)sulfanyl]propanoate was obtained in the same manner as in the fourth step of Example 1 except that methyl 3-[(3-{[t-butyl(diphenyl)silyl]oxy}tetradecyl)sulfanyl]propanoate was used as a starting material.

Fifth step: Methyl 3-({3-[(4-methylbenzene-1-sulfonyl)oxy]tetradecyl}sulfanyl)propanoate was obtained in the same manner as in the fifth step of Example 1 except that methyl 3-[(3-hydroxytetradecyl)sulfanyl]propanoate was used as a starting material.

Sixth step: 3-[(3-[¹⁸F]fluorotetradecyl)sulfanyl]propanoic acid was obtained in the same manner as in the sixth step of Example 1 except that methyl 3-({3-[(4-methylbenzene-1-sulfonyl)oxy]tetradecyl}sulfanyl)propanoate was used as a starting material. The generation of the compound was confirmed by the retention time in HPLC being consistent with the time of non-radiative labeled material shown in Reference Example 4.

### Example 5

Reference Example 12 shows a method for synthesizing 2,2,9,9,10,10-hexamethyl-3,3-diphenyl-5-[(2E)-undec-2-en-1-yl]-4,8-dioxa-3,9-disilaundecane, which is the starting material in the first step.

First step: (5E)-3-{[t-butyl(diphenyl)silyl]oxy}tetradec-5-en-1-ol was obtained in the same manner as in the first step of Example 1 except that 2,2,9,9,10,10-hexamethyl-3,3-diphenyl-5-[(2E)-undec-2-en-1-yl]-4,8-dioxa-3,9-disilaundecane was used as a starting material.

Second step: t-Butyl{[(5E)-1-iodotetradec-5-en-3-yl]oxy}diphenylsilane was obtained in the same manner as in the second step of Example 1 except that (5E)-3-{[t-butyl(diphenyl)silyl]oxy}tetradec-5-en-1-ol was used as a starting material.

Third step: Methyl 3-{[(5E)-3-{[t-butyl(diphenyl)silyl]oxy}tetradec-5-en-1-yl]sulfanyl}propanoate was obtained in the same manner as in the third step of Example 1 except that t-butyl{[(5E)-1-iodotetradec-5-en-3-yl]oxy}diphenylsilane was used as a starting material.

Fourth step: Methyl 3-{[(5E)-3-hydroxytetradec-5-en-1-yl]sulfanyl}propanoate was obtained in the same manner as in the fourth step of Example 1 except that methyl 3-{[(5E)-3-{[t-butyl(diphenyl)silyl]oxy}tetradec-5-en-1-yl]sulfanyl}propanoate was used as a starting material.

Fifth step: Methyl 3-({(5E)-3-[(4-methylbenzene-1-sulfonyl)oxy]tetradec-5-en-1-yl} sulfanyl)propanoate was obtained in the same manner as in the fifth step of Example 1 except that methyl 3-{[(5E)-3-hydroxytetradec-5-en-1-yl]sulfanyl}propanoate was used as a starting material.

Sixth step: 3-{[(5E)-3-[¹⁸F]fluorotetradec-5-en-1-yl]sulfanyl}propanoic acid was obtained in the same manner as in the sixth step of Example 1 except that methyl 3-({(5E)-3-[(4-methylbenzene-1-sulfonyl)oxy]tetradec-5-en-1-yl}sulfanyl)propanoate was used as a starting material. The generation of the compound was confirmed by the retention time in HPLC being consistent with the time of non-radiative labeled material shown in Reference Example 5.

### Example 6

Reference Example 13 shows a method for synthesizing 2,2,9,9,10,10-hexamethyl-5-[(2-octylcyclopropyl)methyl]-3,3-diphenyl-4,8-dioxa-3,9-disilaundecane, which is the starting material in the first step.

First step: 3-{[t-butyl(diphenyl)silyl]oxy}-4-(2-octylcyclopropyl)butan-1-ol was obtained in the same manner as in the first step of Example 1 except that 2,2,9,9,10,10-hexamethyl-5-[(2-octylcyclopropyl)methyl]-3,3-diphenyl-4,8-dioxa-3,9-disilaundecane was used as a starting material.

Second step: t-Butyl{[4-iodo-1-(2-octylcyclopropyl)butan-2-yl]oxy}diphenylsilane was obtained in the same manner as in the second step of Example 1 except that 3-{[t-butyl(diphenyl)silyl]oxy}-4-(2-octylcyclopropyl)butan-1-ol was used as a starting material.

Third step: Methyl 3-{[3-{[t-butyl(diphenyl)silyl]oxy}-4-(2-octylcyclopropyl)butyl]sulfanyl}propanoate was obtained in the same manner as in the third step of Example 1 except that t-butyl{[4-iodo-1-(2-octylcyclopropyl)butan-2-yl]oxy}diphenylsilane was used as a starting material.

Fourth step: Methyl 3-{[3-hydroxy-4-(2-octylcyclopropyl)butyl]sulfanyl}propanoate was obtained in the same manner as in the fourth step of Example 1 except that methyl 3-{[3-{[t-butyl(diphenyl)silyl]oxy}-4-(2-octylcyclopropyl)butyl]sulfanyl}propanoate was used as a starting material.

Fifth step: Methyl 3-({3-[(4-methylbenzene-1-sulfonyl)oxy]-4-(2-octylcyclopropyl)butyl} sulfanyl)propanoate was obtained in the same manner as in the fifth step of Example 1 except that methyl 3-{[3-hydroxy-4-(2-octylcyclopropyl)butyl]sulfanyl}propanoate was used as a starting material.

Sixth step: 3-{[3-[¹⁸F]fluoro-4-(2-octylcyclopropyl)butyl]sulfanyl}propanoic acid was obtained in the same manner as in the sixth step of Example 1 except that methyl 3-({3-[(4-methylbenzene-1-sulfonyl)oxy]-4-(2-octylcyclopropyl)butyl}sulfanyl)propanoate was used as a starting material. The generation of the compound was confirmed by the retention time in HPLC being consistent with the time of non-radiative labeled material shown in Reference Example 6.

### Example 7

Reference Example 14 shows a method for synthesizing 2,2,9,9,10,10-hexamethyl-5-octyl-3,3-diphenyl-4,8-dioxa-3,9-disilaundecane, which is the starting material in the first step.

First step: 3-{[t-butyl(diphenyl)silyl]oxy}undecan-1-ol was obtained in the same manner as in the first step of Example 1 except that 2,2,9,9,10,10-hexamethyl-5-octyl-3,3-diphenyl-4,8-dioxa-3,9-disilaundecane was used as a starting material.

Second step: t-Butyl[(1-iodoundecan-3-yl)oxy]diphenylsilane was obtained in the same manner as in the second step of Example 1 except that 3-{[t-butyl(diphenyl)silyl]oxy}undecan-1-ol was used as a starting material.

Third step: Methyl 5-[(3-{[t-butyl(diphenyl)silyl]oxy}undecyl)sulfanyl]pentanoate was obtained in the same manner as in the third step of Example 1 except that t-butyl[(1-iodoundecan-3-yl)oxy]diphenylsilane was used as a starting material, and methyl 5-sulfanylpentanoate was used instead of methyl 3-sulfanylpropionate.

Fourth step: Methyl 5-[(3-hydroxyundecyl)sulfanyl]pentanoate was obtained in the same manner as in the fourth step of Example 1 except that methyl 5-[(3-{[t-butyl(diphenyl)silyl]oxy}undecyl)sulfanyl]pentanoate was used as a starting material.

Fifth step: Methyl 5-({3-[(4-methylbenzene-1-sulfonyl)oxy]undecyl}sulfanyl)pentanoate was obtained in the same manner as in the fifth step of Example 1 except that methyl 5-[(3-hydroxyundecyl)sulfanyl]pentanoate was used as a starting material.

Sixth step: 5-[(3-[¹⁸F]fluoroundecyl)sulfanyl]pentanoic acid was obtained in the same manner as in the sixth step of Example 1 except that methyl 5-({3-[(4-methylbenzene-1-sulfonyl)oxy]undecyl}sulfanyl)pentanoate was used as a starting material. The generation of the compound was confirmed by the retention time in HPLC being consistent with the time of non-radiative labeled material shown in Reference Example 7.

The chemical structural formulae and physicochemical data of the compounds obtained in each step are shown in Table 1 to Table 6.

**[Table 1]**

| Ex | Str | DAT |
|---|---|---|
| 1-1 | 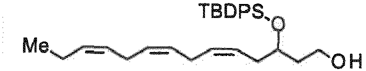 | ESI+: 485[M+Na]⁺ |
| 1-2 | 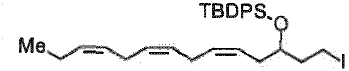 | ESI+: 595[M+Na]⁺ |
| 1-3 | 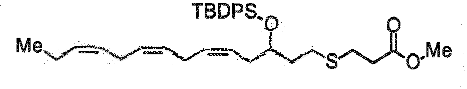 | ESI+: 587[M+Na]⁺ |
| 1-4 | 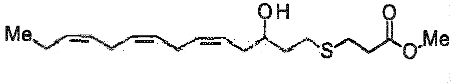 | ESI+: 349[M+Na]⁺ |
| 1-5 | 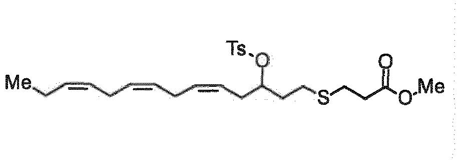 | ESI+: 503[M+Na]⁺ NMR (500 MHz): 7.78-7.83 (2H, m), 7.31-7.36 (2H, m), 5.34-5.51 (3H, m), 5.19-5.33 (3H, m), 4.61-4.70 (1H, m), 3.70 (3H, s), 2.75-2.80 (2H, m), 2.66-2.75 (4H, m), 2.53-2.57 (2H, m), 2.47-2.53 (1H, m), 2.44 (3H, s), 2.33-2.44 (3H, m), 1.98-2.11 (2H, m), 1.77-1.96 (2H, m), 0.98 (3H, t, J = 7.5 Hz) |
| 1-6 | 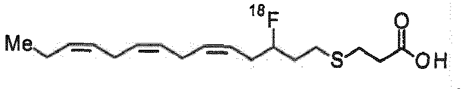 | |
| 2-1 | 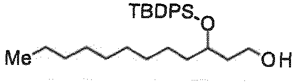 | ESI+: 463[M+Na]⁺ |
| 2-2 | 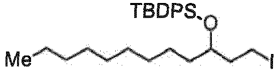 | ESI+: 551 |
| 2-3 | 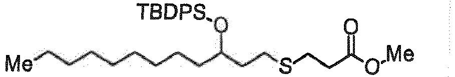 | ESI+: 565[M+Na]⁺ |
| 2-4 | 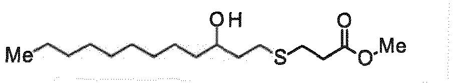 | ESI+: 327[M+Na]⁺ |

**[Table 2]**

| Ex | Str | DAT |
|---|---|---|
| 2-5 | 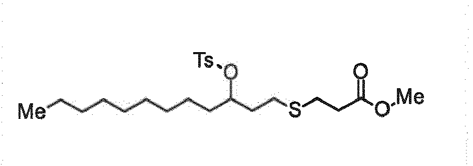 | ESI+: 481[M+Na]⁺ NMR (400 MHz): 7.77-7.82 (2H, m), 7.31-7.37(2H,m), 4.61-4.69 (1H, m), 3.70 (3H, s), 2.67-2.74 (2H, m), 2.54-2.59 (2H, m), 2.36-2.53 (2H, m), 2.45 (3H, s), 1.77-1.96 (2H, m), 1.55-1.62 (1H, m), 1.12-1.33 (15H, m), 0.88 (3H, t, J = 7.0 Hz) |
| 2-6 | 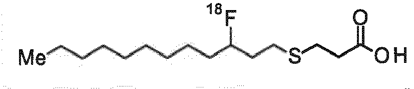 | |
| 3-1 | 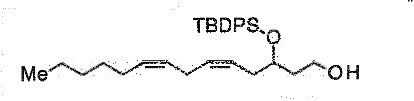 | ESI+: 487[M+Na]⁺ |
| 3-2 | 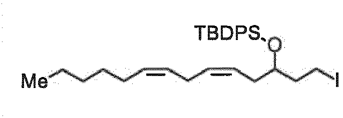 | NMR (500 MHz): 7.65-7.71 (4H, m), 7.35-7.47 (6H, m), 5.30-5.39 (2H, m), 5.16-5.28 (2H, m), 3.75-3.83 (1H, m), 3.10-3.22 (2H, m), 2.50-2.62 (2H, m), 2.14-2.23 (2H, m), 1.94-2.05 (4H, m), 1.22-1.39 (6H, m), 1.06 (9H, s), 0.89 (3H, t, J = 7.0 Hz) |
| 3-3 | 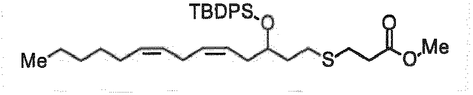 | ESI+: 589[M+Na]⁺ |
| 3-4 | 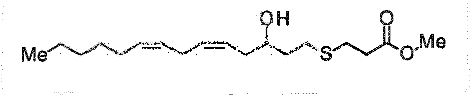 | ESI+: 351[M+Na]⁺ |

**[Table 3]**

| Ex | Str | DAT |
|---|---|---|
| 3-5 | 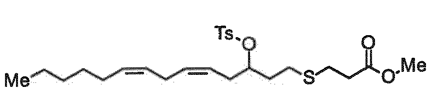 | ESI+: 505[M+Na]⁺ NMR (500 MHz): 7.77-7.83 (2H, m), 7.32-7.37 (2H, m), 5.35-5.47 (2H, m), 5.19-5.29 (2H, m), 4.61-4.69 (1H, m), 3.70 (3H, s), 2.66-2.73 (4H, m), 2.47-2.58 (3H, m), 2.45 (3H, s), 2.32-2.43 (3H, m), 1.98-2.07 (2H, m), 1.77-1.94 (2H, m), 1.24-1.41 (6H, m), 0.89 (3H, t, J = 7.0 Hz) |
| 3-6 | 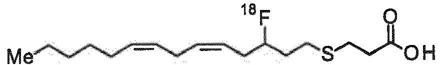 | |
| 4-1 | 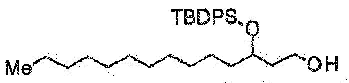 | ESI+: 469 |
| 4-2 | 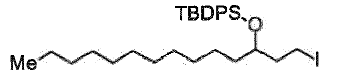 | ESI+: 579 |
| 4-3 | 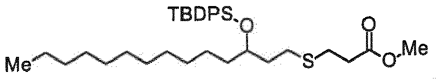 | ESI+: 593[M+Na]⁺ |
| 4-4 | 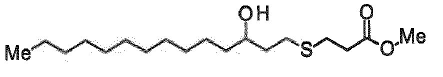 | ESI+: 355[M+Na]⁺ |
| 4-5 | 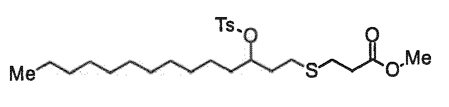 | ESI+: 509[M+Na]⁺ NMR (400 MHz): 7.78-7.82 (2H, m), 7.31-7.36 (2H, m), 4.61-4.69 (1H, m), 3.70 (3H, s), 2.67-2.73 (2H, m), 2.54-2.59 (2H, m), 2.36-2.54 (2H, m), 2.45 (3H, s), 1.77-1.94 (2H, m), 1.50-1.62 (2H, m), 1.13-1.33 (18H, m), 0.89 (3H, t, J = 6.9 Hz) |
| 4-6 | 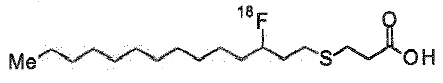 | |

**[Table 4]**

| Ex | Str | DAT |
|---|---|---|
| 5-1 | 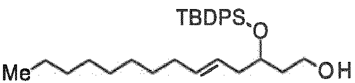 | ESI+: 467 |
| 5-2 | 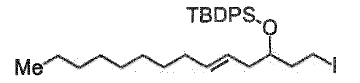 | ESI+: 599[M+Na]⁺ |
| 5-3 | 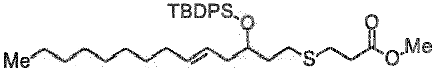 | ESI+: 591[M+Na]⁺ |
| 5-4 | 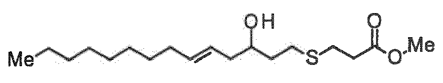 | ESI+: 353[M+Na]⁺ |
| 5-5 | 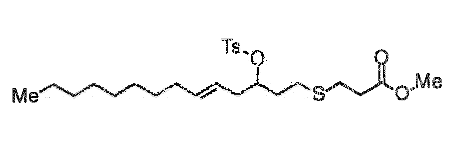 | ESI+: 507[M+Na]⁺ NMR (500 MHz): 7.77-7.81 (2H, m), 7.34 (2H, d, J = 8.0 Hz), 5.39-5.46 (1H, m), 5.14-5.22 (1H, m), 4.60-4.66 (1H, m), 3.70 (3H, s), 2.66-2.72 (2H, m), 2.53-2.58 (2H, m), 2.46-2.53 (1H, m), 2.45 (3H, s), 2.34-2.41 (1H, m), 2.29 (2H, t, J = 6.5 Hz), 1.77-1.94 (4H, m), 1.21-1.32 (12H, m), 0.88 (3H, t, J = 7.0 Hz) |
| 5-6 | 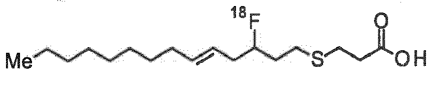 | |
| 6-1 | 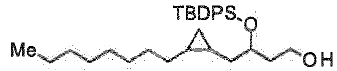 | ESI+: 503[M+Na]⁺ |

**[Table 5]**

| Ex | Str | DAT |
|---|---|---|
| 6-2 | 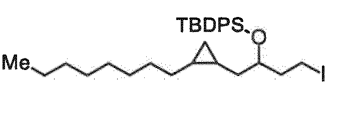 | NMR (500 MHz): 7.66-7.70 (4H, m), 7.34-7.45 (6H, m), 3.77-3.85 (1H, m), 3.17-3.30 (2H, m), 2.01-2.21 (2H, m), 1.54-1.63 (1H, m), 1.14-1.35 (12H, m), 1.04-1.11 (10H, m), 0.75-1.02 (5H, m), 0.48-0.58 (2H, m), 0.38-0.44 (1H, m), -0.38₋-0.74 (1H, m) |
| 6-3 | 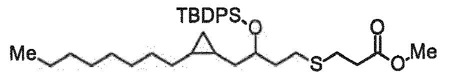 | ESI+: 583 |
| 6-4 | 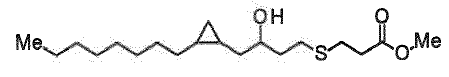 | ESI+: 345 |
| 6-5 | 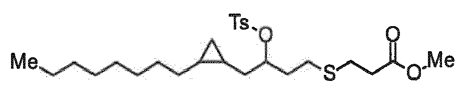 | ESI+: 499 NMR (500 MHz): 7.78-7.83 (2H, m), 7.31-7.36 (2H, m), 4.64-4.73 (1H, m), 3.70 (3H, s), 2.68-2.74 (2H, m), 2.49-2.59 (3H, m), 2.38-2.46 (1H, m), 2.44 (3H, s), 1.88-2.00 (2H, m), 1.70-1.84 (1H, m), 1.23-1.45 (13H, m), 1.12-1.23 (1H, m), 0.93-1.03(1H, m), 0.86-0.92(3H, m), 0.50-0.69 (3H, m), -0.37₋-0.29 (1H, m) |
| 6-6 | 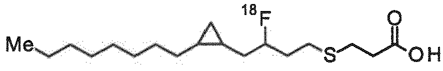 | |
| 7-1 | 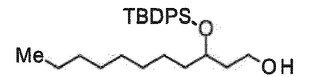 | ESI+: 449[M+Na]⁺ |
| 7-2 | 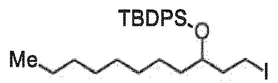 | ESI+: 559[M+Na]⁺ |
| 7-3 | 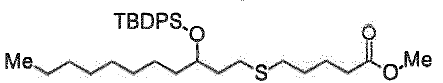 | ESI+: 579[M+Na]⁺ |

**[Table 6]**

| Ex | Str | DAT |
|---|---|---|
| 7-4 | 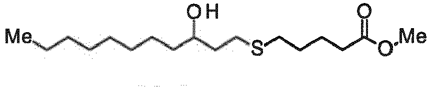 | ESI+: 341[M+Na]⁺ |
| 7-5 | 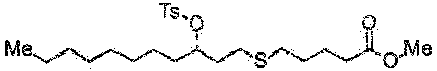 | ESI+: 495[M+Na]⁺ NMR (500 MHz): 7.77-7.82 (2H, m), 7.31-7.36 (2H, m), 4.62-4.69 (1H, m), 3.68 (3H, s), 2.35-2.50 (7H, m), 2.33 (2H, t, J = 7.4Hz), 1.77-1,93 (2H, m), 1.67-1.74 (2H, m), 1.57-1.63 (3H, m) 1.51-1.56 (1H, m), 1.13-1.31 (12H, m), 0.88 (3H, t, J = 7.0 Hz) |
| 7-6 | 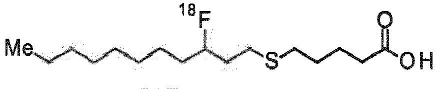 | |

### Reference Example 1

3- {[(5Z,8Z,11Z)-3-fluorotetradeca-5,8,11-trien-1-yl]sulfanyl}propanoic acid used as a standard sample of Example 1 was synthesized. The steps are shown below.

Methyl 3-{[(5Z,8Z,11Z)-3-hydroxytetradeca-5,8,11-trien-1-yl]sulfanyl}propanoate (180 mg) was dissolved in toluene (1.8 mL) under a nitrogen gas stream, PyFluor (98 mg) and DBU (registered trademark) (165 µL) were added at room temperature, and the mixture was stirred for 70 hours. The supernatant was purified by silica gel column chromatography (EtOAc/n-hexane).

THF (1.8 mL) and MeOH (1.8 mL) were added and the obtained residue was dissolved therein, an aqueous sodium hydroxide solution (1M, 1.8 mL) was added at room temperature, and the mixture was stirred for 1 hour. After neutralization by adding an aqueous hydrochloric acid solution (1 M), chloroform and a saturated brine were added, followed by extraction with chloroform to separate the organic layer. The organic layer was dried over magnesium sulfate and concentrated under reduced pressure to obtain 3-{[(5Z,8Z,11Z)-3-fluorotetradeca-5,8,11-trien-1-yl]sulfanyl}propanoic acid (50 mg) as an oily substance.

### Reference Example 2

3-[(3-fluorododecyl)sulfanyl]propanoic acid used as a standard sample of Example 2 was synthesized in the same manner as in Reference Example 1 except that methyl 3-[(3-hydroxydodecyl)sulfanyl]propanoate was used as a starting material.

### Reference Example 3

3-{[(5Z,8Z)-3-fluorotetradeca-5,8-dien-1-yl]sulfanyl}propanoic acid used as a standard sample of Example 3 was synthesized in the same manner as in Reference Example 1 except that methyl 3-{[(5Z,8Z)-3-hydroxytetradeca-5,8-dien-1-yl]sulfanyl}propanoate was used as a starting material.

### Reference Example 4

3-[(3-fluorotetradecyl)sulfanyl]propanoic acid used as a standard sample of Example 4 was synthesized in the same manner as in Reference Example 1 except that methyl 3-[(3-hydroxytetradecyl)sulfanyl]propanoate was used as a starting material.

### Reference Example 5

3-{[(5E)-3-fluorotetradec-5-en-1-yl]sulfanyl}propanoic acid used as a standard sample of Example 5 was synthesized in the same manner as in Reference Example 1 except that methyl 3-{[(5E)-3-hydroxytetradec-5-en-1-yl]sulfanyl}propanoate was used as a starting material.

### Reference Example 6

3-{[3-fluoro-4-(2-octylcyclopropyl)butyl]sulfanyl}propanoic acid used as a standard sample of Example 6 was synthesized in the same manner as in Reference Example 1 except that methyl 3-{[3-hydroxy-4-(2-octylcyclopropyl)butyl]sulfanyl}propanoate was used as a starting material.

### Reference Example 7

5-[(3-fluoroundecyl)sulfanyl]pentanoic acid used as a standard sample of Example 7 was synthesized in the same manner as in Reference Example 1 except that methyl 5-[(3-hydroxyundecyl)sulfanyl]pentanoate was used as a starting material.

### Reference Example 8

2,2,9,9,10,10-hexamethyl-3,3-diphenyl-5-[(2Z,5Z,8Z)-undeca-2,5,8-trien-1-yl]-4,8-dioxa-3,9-disilaundecane, which is a starting material of Example 1 was synthesized. The steps are shown below.

[(3Z,6Z)-nona-3,6-dien-1-yl](triphenyl)phosphanium 4-methylbenzene-1-sulfonate (3.99 g) was dissolved in the mixture of THF (100 mL) and HMPA (6 mL) and cooled to -78°C under a nitrogen gas stream. Potassium hexamethyldisilazide (1 M THF solution, 7.2 mL) was added dropwise, then the mixture was stirred at ice-cooling for 1 hour. The reaction liquid was cooled to -78°C, then a THF (10 mL) solution of 5-{[t-butyl(dimethyl)silyl]oxy}-3-{[t-butyl(diphenyl)silyl]oxy}pentanal (2.00 g) was added dropwise, and the mixture was stirred at the same temperature for 1 hour. After stirring at room temperature for 2 hours, water was added. Ethyl acetate, n-hexane, and water were added, and the organic layer was separated, washed with a saturated brine, dried over magnesium sulfate, and concentrated under reduced pressure. The residue was purified by silica gel column chromatography (EtOAc/n-hexane) to obtain 2,2,9,9,10,10-hexamethyl-3,3-diphenyl-5-[(2Z,5Z,8Z)-undeca-2,5,8-trien-1-yl]-4,8-dioxa-3,9-disilaundecane (2.27 g) as an oily substance.

### Reference Example 9

2,2,9,9,10,10-hexamethyl-5-nonyl-3,3-diphenyl-4,8-dioxa-3,9-disilaundecane, which is a starting material of Example 2 was synthesized. The steps are shown below.

First step: Diethyl ether (10 mL) was added to magnesium (170 mg) under a nitrogen gas stream and the magnesium was suspended, iodine (10 mg) was added, and the mixture was stirred. A small amount of diethyl ether (5 mL) solution of 1-bromononane (1.3 mL) was added dropwise, the mixture was heated until a brown color of the reaction solution disappears, and then all the remaining of the solution was added dropwise. After stirring at room temperature for 15 minutes, the mixture was cooled on an ice bath, and a diethyl ether (5 mL) solution of 3-{[t-butyl(dimethyl)silyl]oxy}propanal (1.0 g) was added dropwise. After stirring at the same temperature for 30 minutes, a saturated aqueous ammonium chloride solution was added. Ethyl acetate and water were added to perform a liquid-separating operation, the organic layer was washed with a saturated brine, dried over magnesium sulfate, and concentrated under reduced pressure. The residue was purified by silica gel column chromatography (EtOAc/n-hexane) to obtain 1-{[t-butyl(dimethyl)silyl]oxy}dodecan-3-ol (1.27 g) as an oily substance.

Second step: 1-{[t-butyl(dimethyl)silyl]oxy}dodecan-3-ol (1.27 g) was dissolved in DMF (2.5 mL) under a nitrogen gas stream, imidazole (0.82 g) and t-butyldiphenylchlorosilane (1.45 mL) were added, and the mixture was stirred at room temperature for 70 hours. Ethyl acetate and water were added to perform a liquid-separating operation, the organic layer was washed with a saturated brine, dried over magnesium sulfate, and then concentrated under reduced pressure. The residue was purified by silica gel column chromatography (EtOAc/n-hexane) to obtain 2,2,9,9,10,10-hexamethyl-5-nonyl-3,3-diphenyl-4,8-dioxa-3,9-disilaundecane (2.08 g) as an oily substance.

### Reference Example 10

2,2,9,9,10,10-hexamethyl-3,3-diphenyl-5-[(2Z,5Z)-undeca-2,5-dien-1-yl]-4,8-dioxa-3,9-disilaundecane used as starting material of Example 3 was synthesized in the same manner as in Reference Example 8 except that [(3Z)-non-3-en-1-yl](triphenyl)phosphanium 4-methylbenzene-1-sulfonate was used instead of [(3Z,6Z)-nona-3,6-dien-1-yl](triphenyl)phosphanium 4-methylbenzene-1-sulfonate as a starting material.

### Reference Example 11

2,2,9,9,10,10-hexamethyl-3,3-diphenyl-5-undecyl-4,8-dioxa-3,9-disilaundecane used as starting material of Example 4 was synthesized in the same manner as in Reference Example 9 except that 1-bromoundecane was used instead of 1-bromononane as a starting material.

### Reference Example 12

2,2,9,9,10,10-hexamethyl-3,3-diphenyl-5-[(2E)-undec-2-en-1-yl]-4,8-dioxa-3,9-disilaundecane used as starting material of Example 5 was synthesized. The steps are shown below.

Lithium bromide (0.55 g) was put in a reaction vessel, reduced pressure by a vacuum pump with heating on an oil bath at 150°C, and dried for 1 hour. After open cooling, THF (15 mL) was added, subsequently nonyl(triphenyl)phosphonium bromide (1.5 g) was added, and the mixture was stirred at room temperature for 10 minutes. After cooling to -78°C, phenyl lithium (2.0 M dibutyl ether solution, 1.6 mL) was added dropwise. After stirring at room temperature for 30 minutes, the mixture was cooled again to -78°C. A THF (7.5 mL) solution of 5-{[t-butyl(dimethyl)silyl]oxy}-3-{[t-butyl(diphenyl)silyl]oxy}pentanal (1.5 g) was added dropwise, then phenyl lithium (2.0 M dibutyl ether solution, 1.6 mL) was added dropwise, and the mixture was stirred at -78°C for 30 minutes. The mixture was further stirred for 30 minutes without a bath. The mixture was cooled again to -78°C, and hydrogen chloride (1 M diethyl ether solution, 3.19 mL) was added. Potassium-t-butoxide (0.40 g) was added, and the mixture was stirred for 1.5 hours without a bath. Water was added to the reaction solution, and then ethyl acetate was added to perform a liquid-separating operation. The organic layer was washed with a saturated brine, dried over magnesium sulfate, and then concentrated under reduced pressure. The residue was purified by silica gel column chromatography (EtOAc/n-hexane) to obtain 2,2,9,9,10,10-hexamethyl-3,3-diphenyl-5-[(2E)-undec-2-en-1-yl]-4,8-dioxa-3,9-disilaundecane (1.37 g) as an oily substance.

### Reference Example 13

2,2,9,9,10,10-hexamethyl-5-[(2-octylcyclopropyl)methyl]-3,3-diphenyl-4,8-dioxa-3,9-disilaundecane used as starting material of Example 6 was synthesized. The steps are shown below.

First step: 2-[2-(4-methoxyphenyl)-1,3-dioxan-4-yl]ethan-1-ol (3.76 g) was dissolved in dichloromethane (40 mL) under a nitrogen gas stream, imidazole (1.50 g) and t-butyldimethylsilyl chloride (2.62 g) were added at room temperature, and the mixture was stirred for 30 minutes. After adding aqueous sodium bicarbonate, water was added, followed by extraction with chloroform, the extract was dried over magnesium sulfate, and then concentrated under reduced pressure. The residue was purified by silica gel column chromatography (EtOAc/n-hexane) to obtain t-butyl{2-[2-(4-methoxyphenyl)-1,3-dioxan-4-yl]ethoxy}dimethylsilan (5.44 g) as an oily substance.

Second step: t-Butyl{2-[2-(4-methoxyphenyl)-1,3-dioxan-4-yl]ethoxy}dimethylsilan (5.44 g) was dissolved in toluene (20 mL) under a nitrogen gas stream and ice-cooled. Aluminum diisobutyl hydride (1 M toluene solution, 18.5 mL) was added dropwise, and the mixture was stirred for 3 hours while heating to room temperature. After ice-cooling again, aluminum diisobutyl hydride (1 M toluene solution, 9 mL) was added, and the mixture was stirred further for 3 hours. After adding water, an aqueous sodium potassium tartrate solution was added until the mixture can be stirred. Ethyl acetate was added to perform a liquid-separating operation, the organic layer was washed with a saturated brine, dried over magnesium sulfate, and then concentrated under reduced pressure. The residue was purified by silica gel column chromatography (EtOAc/n-hexane) to obtain 5-{[t-butyl(dimethyl)silyl]oxy}-3-[(4-methoxyphenyl)methoxy]pentan-1-ol (1.88 g) as an oily substance.

Third step: 5- {[t-butyl(dimethyl)silyl]oxy}-3-[(4-methoxyphenyl)methoxy]pentan-1-ol (1.88 g) was dissolved in dichloromethane (40 mL), and then ice-cooled, Dess-Martin reagent (2.25 g) was added little by little, and the mixture was stirred at the same temperature for 1 hour. An aqueous sodium thiosulfate solution and aqueous sodium bicarbonate were added, followed by extraction with chloroform. The organic layer was dried over magnesium sulfate, and then concentrated under reduced pressure. The residue was purified by silica gel column chromatography (EtOAc/n-hexane) to obtain 5-{[t-butyl(dimethyl)silyl]oxy}-3-[(4-methoxyphenyl)methoxy]pentanal (1.61 g) as an oily substance.

Fourth step: t-Butyl({(5Z)-3-[(4-methoxyphenyl)methoxy]tetradec-5-en-1-yl}oxy)dimethylsilane was synthesized in the same manner as in Reference Example 8 except that nonyl(triphenyl)phosphanium bromide and 5-{[t-butyl(dimethyl)silyl]oxy}-3-[(4-methoxyphenyl)methoxy]pentanal were used instead of [(3Z,6Z)-nona-3,6-dien-1-yl](triphenyl)phosphanium 4-methylbenzene-1-sulfonate as a starting material and 5-{[t-butyl(dimethyl)silyl]oxy}-3-{[t-butyl(diphenyl)silyl]oxy}pentanal, respectively.

Fifth step: t-Butyl({(5Z)-3-[(4-methoxyphenyl)methoxy]tetradec-5-en-1-yl}oxy)dimethylsilane (1.95 g) was dissolved in dichloromethane (59 mL), water (3.3 mL) was added, the mixture was stirred, and then DDQ (1.24 g) was added at room temperature, and the mixture was stirred at the same temperature for 30 minutes. n-Hexane was added, and solid substrate was filtered out. Water was added to the filtrate, followed by extraction with chloroform, the organic layer was dried over magnesium sulfate, and then concentrated under reduced pressure. The residue was purified by silica gel column chromatography (chloroform) to obtain (5Z)-1-{[t-butyl(dimethyl)silyl]oxy}tetradec-5-en-3-ol (1.31 g) as an oily substance.

Sixth step: (5Z)-1-{[t-butyl(dimethyl)silyl]oxy}tetradec-5-en-3-ol (1.31 g) was dissolved in dichloromethane (65 mL) under a nitrogen gas stream and ice-cooled. Diethyl zinc (1 M n-hexane solution, 19 mL) was added, the mixture was stirred at the same temperature for 5 minutes, and then diiodomethane (1.54 mL) was added. After stirring at the same temperature for 30 minutes, the mixture was warmed up naturally as it was, and stirred at room temperature for 1 hour. The mixture was ice-cooled again, and a saturated aqueous ammonium chloride solution was added, followed by extraction with chloroform. The organic layer was dried over magnesium sulfate, and then concentrated under reduced pressure. The residue was purified by silica gel column chromatography (EtOAc/n-hexane) to obtain 4-{[t-butyl(dimethyl)silyl]oxy}-1-(2-octylcyclopropyl)buthan-2-ol (1.33 g) as an oily substance.

Seventh step: 2,2,9,9,10,10-hexamethyl-5-[(2-octylcyclopropyl)methyl]-3,3-diphenyl-4,8-dioxa-3,9-disilaundecane was synthesized in the same manner as in the second step of Reference Example 9 except that 4-{[t-butyl(dimethyl)silyl]oxy}-1-(2-octylcyclopropyl)buthan-2-ol was used instead of 1-{[t-butyl(dimethyl)silyl]oxy}dodecan-3-ol as a starting material.

### Reference Example 14

2,2,9,9,10,10-hexamethyl-5-octyl-3,3-diphenyl-4,8-dioxa-3,9-disilaundecane used as starting material of Example 7 was synthesized. The steps are shown below.

First step: Diethyl ether (10 mL) was added to 3-{[t-butyl(dimethyl)silyl]oxy}propanal (1.16 g) under a nitrogen gas stream and cooled on an ice bath, octyl magnesium bromide (2 M diethyl ether solution, 3.4 mL) was added dropwise, and the mixture was stirred at the same temperature for 90 minutes. A saturated aqueous ammonium chloride solution and water were added to reaction solution, ethyl acetate was added to perform a liquid-separating operation. The organic layer was washed with a saturated brine, dried over magnesium sulfate, and then concentrated under reduced pressure. The residue was purified by silica gel column chromatography (EtOAc/n-hexane) to obtain 1-{[t-butyl(dimethyl)silyl]oxy}undecan-3-ol (0.72 g) as an oily substance.

Second step: 2,2,9,9,10,10-hexamethyl-5-octyl-3,3-diphenyl-4,8-dioxa-3,9-disilaundecane was synthesized in the same manner as in the second step of Reference Example 9 except that 1-{[t-butyl(dimethyl)silyl]oxy}undecan-3-ol was used instead of 1-{[t-butyl(dimethyl)silyl]oxy}dodecan-3-ol as a starting material.

### Reference Example 15

[¹⁸F]FTO was obtained using the same method as the method described in J. Nucl. Med., 2010, 51 (8), 1310-1317, and the solution was prepared in the same manner as in the sixth step of Example 1.

The chemical structural formulae and physicochemical data of the compounds obtained in Reference Examples 1 to 15 are shown in Table 7 to Table 9.

**[Table 7]**

| Ref | Str | DAT |
|---|---|---|
| 1 | 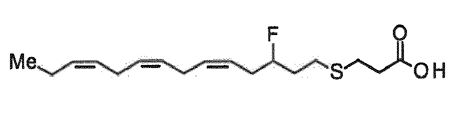 | ESI+: 315 NMR (400 MHz): 5.49-5.58 (1H, m), 5.26-5.48 (5H, m), 4.53-4.73 (1H, m), 2.58-2.86 (10H, m), 2.31-2.54 (2H, m), 2.02-2.12 (2H, m), 1.72-2.02 (2H, m), 0.98 (3H, t, J = 7.6 Hz) |
| 2 | 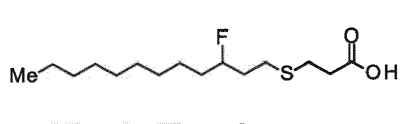 | ESI-: 291 NMR (400 MHz): 4.47-4.68 (1H, m), 2.78 (2H, t, J = 7.2 Hz), 2.57-2.75 (4H, m), 1.37-1.99 (6H, m), 1.20-1.38 (12H, m), 0.88 (3H, t, J = 6.8 Hz) |
| 3 | 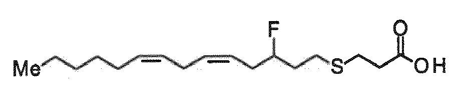 | ESI-: 315 NMR (500 MHz): 5.49-5.59 (1H, m), 5.37-5.45 (2H, m), 5.28-5.35 (1H, m), 4.55-4.71 (1H, m), 2.59-2.84 (8H, m), 2.32-2.53 (2H, m), 2.01-2.08 (2H, m), 1.74-2.01 (2H, m), 1.22-1.39 (6H, m), 0.89 (3H, t, J = 7.0 Hz) |
| 4 | 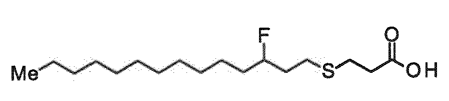 | ESI-: 319 NMR (500 MHz): 4.49-4.67 (1H, m), 2.77-2.82 (2H, m), 2.58-2.75 (4H, m), 1.86-1.98 (1H, m), 1.60-1.86 (2H, m), 1.40-1.60 (2H, m), 1.22-1.36 (17H, m), 0.88 (3H, t, J = 7.0 Hz) |

**[Table 8]**

| Ref | Str | DAT |
|---|---|---|
| 5 | 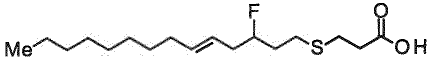 | ESI-: 317 NMR (500 MHz): 5.49-5.57 (1H, m), 5.34-5.43 (1H, m), 4.52-4.67 (1H, m), 2.77-2.82 (2H, m), 2.58-2.75 (4H, m), 2.23-2.43 (2H, m), 1.97-2.04 (2H, m), 1.73-1.97 (2H, m), 1.21-1.39 (12H, m), 0.88 (3H, t, J = 7.0 Hz) |
| 6 | 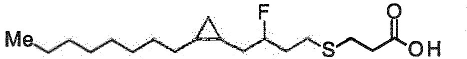 | ESI-: 331 NMR (500 MHz): 4.59-4.75 (1H, m), 2.81 (2H, t, J = 7.3 Hz), 2.71-2.77 (1H, m), 2.60-2.70 (3H, m), 1.79-2.03 (3H, m), 1.49-1.76 (1H, m), 1.22-1.42 (13H, m), 1.08-1.19 (1H, m), 0.88 (3H, t, J = 7.0 Hz), 0.70-0.84 (2H, m), 0.64-0.70 (1H, m), -0.27₋-0.18 (1H, m) |
| 7 | 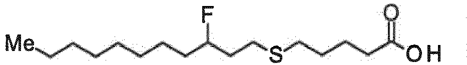 | ESI-: 305 NMR (500 MHz): 4.49-4.67 (1H, m), 2.52-2.70 (4H, m), 2.39 (2H, t, J=7.3 Hz), 1.85-1.98 (1H, m), 1.61-1.84 (6H, m), 1.40-1.60 (2H, m), 1.21-1.40 (11H, m), 0.88 (3H, t, J = 7.2 Hz) |
| 8 | 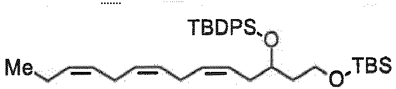 | NMR (500 MHz): 7.65-7.71 (4H, m), 7.28-7.43 (6H, m), 5.19-5.43 (6H, m), 3.87-3.97 (1H, m), 3.56-3.71 (2H, m), 2.67-2.77 (2H, m), 2.53-2.65 (2H, m), 2.13-2.24 (2H, m), 1.94-2.11 (2H, m), 1.64-1.76 (2H, m), 1.03-1.07 (9H, m), 0.97 (3H, t, J = 7.6 Hz), 0.84 9H, s), -0.04-0.01 (6H, m) |
| 9 | 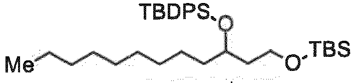 | ESI+: 555 |
| 10 | 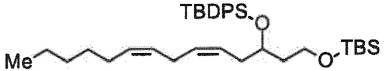 | ESI+: 579 |

**[Table 9]**

| Ref | Str | DAT |
|---|---|---|
| 11 | 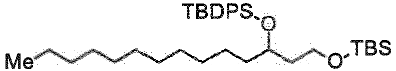 | CI+: 583 |
| 12 | 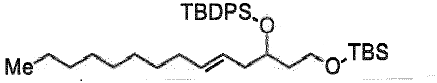 | ESI+: 581 |
| 13 | 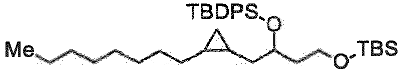 | ESI+: 595 |
| 14 | 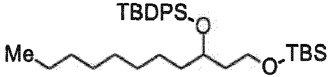 | ESI+: 563[M+Na]⁺ |
| 15 | 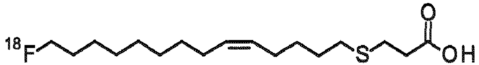 | |

### Example 8

### PET Test Using Normal Cynomolgus Monkey

### (Experimental Method)

Solutions containing each compound prepared in Examples 1 to 7 and Reference Example 15 ([¹⁸F]FTO) were used to perform a heart contrast PET test using normal cynomolgus monkeys.

For performing non-invasive PET imaging, in a state in which a male cynomolgus monkey was anesthetized with inhalation anesthetic isoflurane, the monkey was held at a supine body position on a PET camera SHR17000 (manufactured by Hamamatsu Photonics K.K.), the solution containing each compound (about 300 MBq) was administered from the lower extremity vein, and continuous imaging was performed for 180 minutes or more after the administration. The PET images were reconstructed by dynamic row-action maximum likelihood algorithm (DRAMA) and average images per each time section which is divided every 20 minutes from 0 minutes to 180 minutes after the administration, are obtained. Then, the region of interest (ROI) was set in the myocardium region or bone (near the spine), and the accumulation (standardized uptake value (SUV)) of each compound was calculated as {radioactivity count in the ROI (MBq/cc)/[dose (MBq)/body weight (g)]}.

### (Result)

The SUV values quantified by setting the ROI for the myocardium and the bone of each compound are shown in Tables 10 and 11 as SUV (myocardium) and SUV (bone). In the Tables, Examples 1 to 7 show the value of 1 case, Reference Example 15 shows the average ± standard error of 3 cases, respectively. A case where the target organ cannot be confirmed in the image and the ROI cannot be set is denoted as n.d. The time in the Tables represents an end point of each time section, for example, "20 minutes" represents the SUV value from 0 minutes to 20 minutes after, and "180 minutes" represents the SUV value from 160 minutes to 180 minutes after the administration. In addition, Maximum Intensity Projection (MIP) PET images 60 minutes and 180 minutes after the administration of the compounds of Reference Example 15 ([¹⁸F]FTO) and Example 6 are shown in FIG. 1.

**[Table 10]**

| SUV (myocardium) | 20 minutes | 60 minutes | 120 minutes | 180 minutes |
|---|---|---|---|---|
| Reference Example 15 | 6.45 ± 0.59 | 6.54 ± 0.51 | 5.90 ±0.58 | 5.21 ± 0.59 |
| Example 1 | 4.87 | 4.13 | 3.21 | 2.80 |
| Example 2 | 7.40 | 7.53 | 6.74 | 5.92 |
| Example 3 | 5.75 | 3.20 | 2.80 | 2.41 |
| Example 4 | 6.65 | 5.44 | 4.38 | 3.92 |
| Example 5 | 6.84 | 6.13 | 5.16 | 5.09 |
| Example 6 | 7.74 | 6.24 | 4.80 | 3.98 |
| Example 7 | 11.6 | 11.4 | 10.0 | 8.44 |

**[Table 11]**

| SUV (bone) | 20 minutes | 60 minutes | 120 minutes | 180 minutes |
|---|---|---|---|---|
| Reference Example 15 | 1.42 ± 0.10 | 2.27 ± 0.13 | 13.82 ± 0.60 | 5.05 ± 0.99 |
| Example 1 | 0.87 | 0.69 | 0.75 | 0.79 |
| Example 2 | 1.40 | 1.05 | 1.34 | 1.14 |
| Example 3 | 2.12 | 2.00 | 2.07 | 2.08 |
| Example 4 | 1.57 | 1.13 | 1.27 | 1.51 |
| Example 5 | 1.45 | 1.36 | 1.28 | 1.40 |
| Example 6 | n.d. | n.d. | n.d. | n.d. |
| Example 7 | 1.87 | 3.41 | 5.11 | 6.36 |

From the results in Table 10 and Table 11, it was shown that the ratios of the accumulation to the myocardium with respect to the non-specific accumulation to the bone in Examples 1 to 7 are improved compared to Reference Example 15 ([¹⁸F]FTO). In particular, the non-specific accumulation of Example 6 to the bone was not detected at all, and as shown in FIG. 1, excellent heart PET imaging could be performed.

From these results, it was confirmed that the compounds of Examples of the present invention in which ¹⁸F was substituted at a specific substitution position show higher heart accumulation compared to non-specific accumulation to the bone.

### Industrial Applicability

The labeled fatty acid derivative of the present invention can be used as a radiolabeled tracer for rapid and non-invasive sorting of heart disease patients, diagnosis of the therapeutic effect of a therapeutic drug for heart disease, and the like.

## Claims

1. A labeled fatty acid derivative represented by a formula (I) or a salt thereof, 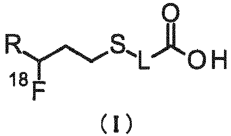 (in the formula,
L is C₂₋₄ alkylene,
R is C₆₋₁₄ alkyl, the C₆₋₁₄ alkyl may have one to three double bonds, and three continuous carbon atoms in the C₆₋₁₄ alkyl may integrally form a cyclopropane ring,
provided that, a case where R is (2Z)-undec-2-en-1-yl group is excluded).

2. The labeled fatty acid derivative or salt thereof according to claim 1,
wherein L is ethylene.

3. The labeled fatty acid derivative or salt thereof according to claim 2,
wherein R is C₆₋₁₄ alkyl, and
three continuous carbon atoms in the C₆₋₁₄ alkyl integrally form a cyclopropane ring.

4. The labeled fatty acid derivative or salt thereof according to claim 1,
wherein the labeled fatty acid derivative is a labeled fatty acid derivative selected from the group consisting of:
3-{[(5Z,8Z,11Z)-3-[¹⁸F]fluorotetradeca-5,8,11-trien-1-yl]sulfanyl}propanoic acid, 3-[(3-[¹⁸F]fluorododecyl)sulfanyl]propanoic acid,
3-{[(5Z,8Z)-3-[¹⁸F]fluorotetradeca-5,8-dien-1-yl]sulfanyl}propanoic acid,
3 -[(3-[¹⁸F]fluorotetradecyl)sulfanyl]propanoic acid,
3-{[(5E)-3-[¹⁸F]fluorotetradec-5-en-1-yl]sulfanyl}propanoic acid,
3-{[3-[¹⁸F]fluoro-4-(2-octylcyclopropyl)butyl] sulfanyl}propanoic acid, and
5-[(3-[¹⁸F]fluoroundecyl)sulfanyl]pentanoic acid.

5. A composition for diagnostic imaging comprising:
the labeled fatty acid derivative or salt thereof according to any one of claims 1 to 4; and
a pharmaceutically acceptable carrier.

6. The composition for diagnostic imaging according to claim 5,
wherein the composition for diagnostic imaging is a composition for diagnostic imaging of heart disease.

7. The composition for diagnostic imaging according to claim 6,
wherein the heart disease is an ischemic heart disease.

8. Use of the labeled fatty acid derivative or salt thereof according to any one of claims 1 to 4, for production of a composition for diagnostic imaging of heart disease.

9. The use of the labeled fatty acid derivative or salt thereof according to claim 8,
wherein the heart disease is an ischemic heart disease.

10. Use of the labeled fatty acid derivative or salt thereof according to any one of claims 1 to 4, for diagnostic imaging of heart disease.

11. The use of the labeled fatty acid derivative or salt thereof according to claim 10,
wherein the heart disease is an ischemic heart disease.

12. The labeled fatty acid derivative or salt thereof according to any one of claims 1 to 4, for use in diagnostic imaging of heart disease.

13. The labeled fatty acid derivative or salt thereof according to claim 12,
wherein the heart disease is an ischemic heart disease.

14. A method for diagnostic imaging of heart disease comprising:
administering a detectable amount of the labeled fatty acid derivative or salt thereof according to any one of claims 1 to 4 to a subject.

15. The method for diagnostic imaging according to claim 14,
wherein the heart disease is an ischemic heart disease.

16. A compound represented by a formula (1g) or a salt thereof, 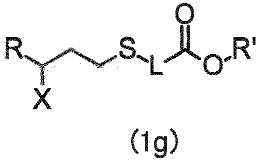 (in the formula,
L is C₂₋₄ alkylene,
R is C₆₋₁₄ alkyl, the C₆₋₁₄ alkyl may have one to three double bonds, three continuous carbon atoms in the C₆₋₁₄ alkyl may integrally form a cyclopropane ring,
R' is H or a lower alkyl group which may be substituted, and
X is a leaving group,
provided that, a case where R is (2Z)-undec-2-en-1-yl group is excluded).

17. The compound or salt thereof according to claim 16,
wherein R' is a lower alkyl which may be substituted.

18. The compound or salt thereof according to claim 17,
wherein L is ethylene.

19. The compound or salt thereof according to claim 18,
wherein R is C₆₋₁₄ alkyl, and
three continuous carbon atoms in the C₆₋₁₄ alkyl integrally form a cyclopropane ring.

20. A method for producing the labeled fatty acid derivative or salt thereof according to any one of claims 1 to 4, the method comprising:
allowing a [¹⁸F]fluoride ion to react with the compound or salt thereof according to claim 16.

21. A kit for preparing the composition for diagnostic imaging according to claim 5, comprising:
the compound or salt thereof according to any one of claims 16 to 19.
